# NEW EUROPEAN PATENT SPECIFICATION

(11) **EP 1 306 069 B2**
(45) Date of publication and mention of the opposition decision: **12.02.2014**
(45) Mention of the grant of the patent: 11.08.2010
(21) Application number: 02257139.2
(22) Date of filing: 15.10.2002
(51) Int. Cl.: A61F 13/84, A61F 13/47, A61F 13/42, A61F 13/15

(54) **Absorbent article and package thereof**
Absorbierender Artikel und dazugehörige Verpackung
Article absorbant et son emballage

(30) Priority: 23.10.2001 JP 2001324811
(43) Date of publication of application: 02.05.2003
(73) Proprietor: UNI-CHARM CORPORATION, Shikokuchuo-shi, Ehime-ken (JP)
(72) Inventor: Wada, Mitsuhiro, c/o Technical Center, Mitoyo-gun, Kagawa-ken 769-1602 (JP); Suga, Ayami, c/o Technical Center, Mitoyo-gun, Kagawa-ken 769-1602 (JP)
(74) Representative: Eke, Philippa Dianne

(56) References cited:
- EP-A- 0 685 214
- EP-A- 1 153 838
- US-A1- 2002 062 114
- US-B1- 6 235 964
- US-B1- 6 284 942

## Description

### BACKGROUND OF THE INVENTION

### Field of the Invention

The present invention relates to an absorbent article with which a user can understand a relationship between a liquid absorption capacity of the article and an amount of discharged liquid.

### Description of the Related Art

Sanitary napkins, vaginal discharge absorbing sheets (panty liners), urine absorbing pads for patients suffering from incontinence (incontinence pads) and disposable diapers are known as absorbent articles for absorbing liquids discharged from the human body. Absorbent articles of this kind are constructed such that a liquid permeable topsheet is disposed on a body surface of an absorbent layer so that discharged liquids given to the topsheet can be absorbed and retained by the absorbent layer. For each absorbent article, a manufacturer provides various kinds of products that differ in length and thickness of the absorbent layer, so that a user can choose one having the most appropriate size depending on how much liquid will be discharged.

For example, a sanitary napkin is divided into three main classes: " small" , " medium" and " large" depending on the length of the absorbent layer, and subdivided into three subclasses: " slim" , " normal" and " heavy" depending on the thickness of the absorbent layer. A discharge amount of menstrual blood during a woman's menstruation varies depending on individual menstrual cycle, individual physical constitution, individual physique and so on. Therefore, each user has to choose one that is most suitable for use among the various kinds of products, while taking into consideration her physical constitution, menstrual cycle or wearing time.

Also in the case of panty liners, incontinence pads and disposable diapers, users have to choose ones suitable for use, because discharge amounts vary depending on age, physique, physical condition during use and so on.

For each absorbent article, as set forth above, a manufacturer provides various kinds of products that differ in liquid absorption capacity by changing length and/or thickness of the absorbent layer. However, actual users usually do not have clear criteria for choosing a product that is suitable for use.

For example, a sanitary napkin user is often unable to make a decision which grade is most suitable for use among various kinds of products, at the time of purchase or use. In general, she tends to choose and use a sanitary napkin having an absorbent layer that is excessively long or thick, because of fear that a menstrual blood might leak laterally from a sanitary napkin. When worn, such an oversized sanitary napkin will unnecessarily interfere with motions of the body of a wearer.

In addition, even if a sanitary napkin that has been used heretofore does not suit her constitution, physique and so on and she wants to use a product of a different kind, she is not certain about a relationship between an absorption capacity of the heretofore used sanitary napkin and an absorption capacity of a new sanitary napkin that she wants to use. Therefore, she may be unable to make a decision which grade should be chosen for the sanitary napkin that she wants to use.

The same statements are true for the case of panty liners, incontinence pads and disposable diapers. That is, it is difficult to choose an absorbent article having a liquid absorption capacity suited to user's physical condition and so on, resulting in purchasing an oversized product. It is also difficult to choose a suitable product when a user wants to change over from a heretofore used product to a product of a different kind.

Further prior art arrangements are known from US 6,284,942 and EP 0 685 214. US 6,284,942 discloses an incontinence pad provided with a plurality of colour changing zones and EP 0 685 214 discloses an absorbent article comprising a top layer and a fibrous layer bonded together at a plurality of fusion spots.

### SUMMARY OF THE INVENTION

The present invention has been worked out in view of the shortcoming in the prior art set forth above. It is therefore an object of the present invention to provide an absorbent article with which a user can measure an extent of liquid spread immediately after use, facilitating subsequent choice of product.

According to the present invention, there is provided an absorbent article as recited in Claim 1.

After use of the absorbent article, a user can measure an extent of liquid spread by comparing the absorbed and spread liquid with the reference mark. Therefore, the user can understand a relationship between user's physical condition and an amount of discharged liquid, a relationship between wearing time and an amount of discharged liquid, and so on. In case where the absorbent article is a sanitary napkin, for example, a user can understand relationships between an amount of discharged liquid and her menstrual cycle, physical constitution, physical condition, wearing time, and so on. With such data, the user can understand her physical condition, an appropriate wearing time, and so on. Moreover, she can use the data as criteria for choosing a product when she is in the same physical condition again.

A plurality of reference marks are provided in the liquid receiving region such that one reference mark is spaced apart from another reference mark toward a periphery of the liquid receiving region. In this case, an amount of discharged liquid after use of the absorbent article can be measured on a step-by-step basis.

Preferably, the reference mark is a continuous or discontinuous border line surrounding a predetermined area of the liquid receiving region. In this case, an amount of discharged liquid that is absorbed and spread in the absorbent article can be measured from a spread area. However, the reference mark should not be limited to such a border line, but may be provided such that a predetermined length of reference lines linearly extending in a transverse direction of the absorbent layer are spaced apart from each other in a longitudinal direction of the absorbent layer, for instance.

Preferably, a grade designator assigning a grade to the reference mark is provided on the body surface of the topsheet along with the reference mark. The term " grade designator" as used herein means any of numerical character, letter and symbol. In this case, an amount of discharged liquid within wearing time can be graded by liquid spread after use.

Preferably, the body surface of the topsheet is colored or patterned differently between a region surrounded by the reference mark and a region surrounding the reference mark. With the different regions divided by the reference mark being differently colored or patterned, the absorbent article can improve in appearance. In addition, the different regions can be clearly visually distinguished from each other.

With the density of the absorbent layer being thus changed, a discharged liquid can be sufficiently spread in the region surrounded by the reference mark, before it goes beyond the reference mark to spread to the region surrounding the reference mark, which facilitates the measurement of an extent of liquid spread based on the reference mark.

### BRIEF DESCRIPTION OF THE DRAWINGS

The present invention will be understood more fully from the detailed description given hereinafter and from the accompanying drawings of the preferred embodiments of the present invention, which, however, should not be taken to be limitative to the invention, but are for explanation and understanding only.

In the drawings:
Fig. 1 is a top plan view showing a medium size sanitary napkin as an absorbent article according to one example not comprising an embodiment of the present invention;
Fig. 2 is a sectional view taken along line II - II of Fig. 1;
Fig. 3 is a sectional view which is taken along line II - II as well, but shows another structure which constitutes a first embodiment of the present invention;
Fig. 4 is a top plan view showing a large size sanitary napkin as an absorbent article according to another embodiment of the present invention;
Fig. 5 is a perspective view showing a package of sanitary napkins;
Figs. 6A and 6B illustrate indications to be printed on the package;
Fig. 7 is a top plan view showing an absorbent article according to still another example not comprising an embodiment of the present invention;
Fig. 8 is a top plan view showing an absorbent article according to still another example not comprising an embodiment of the present invention; and
Fig. 9 is a perspective view showing an absorbent article according to an example which does not constitute an embodiment of the present invention.

### DESCRIPTION OF THE PREFERRED EMBODIMENT

The present invention will be discussed hereinafter in detail in terms of the preferred embodiment according to the present invention with reference to the accompanying drawings. In the following description, numerous specific details are set forth in order to provide a thorough understanding of the present invention. It will be obvious, however, to those skilled in the art that the present invention may be practiced without these specific details. In other instance, well-known structures are not shown in detail in order to avoid unnecessary obscurity of the present invention.

Throughout the disclosure and appended claims, it should be noted that absorbent articles and components thereof, including topsheet, liquid barrier sheet, absorbent layer, and so on, have a body surface and a garment surface. As used herein, " body surface" means that surface of the article or components which is intended to be worn toward or adjacent to the body of the wearer, while the " garment surface" is on the opposite side and is intended to be worn toward or placed adjacent to an undergarment if the absorbent article is worn inside the undergarment.

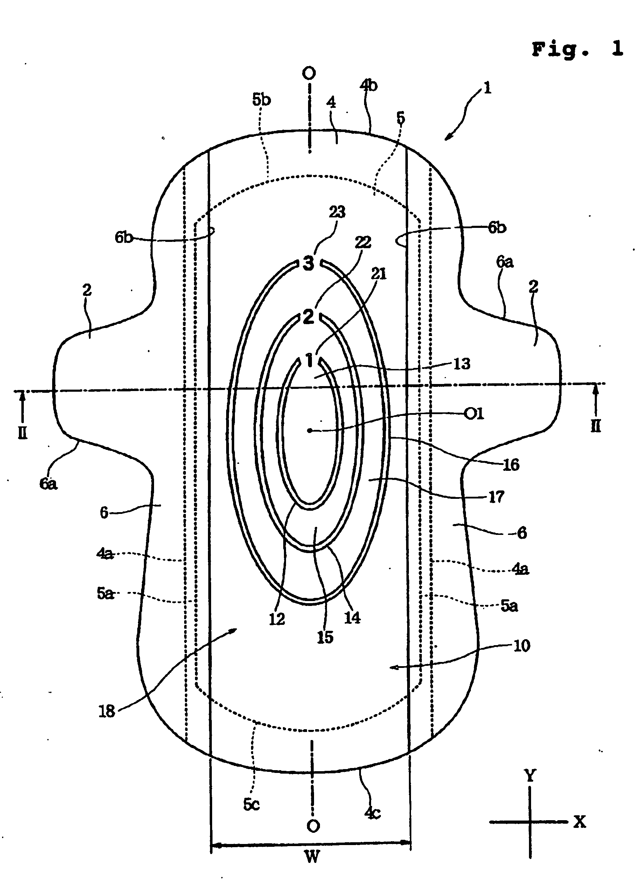

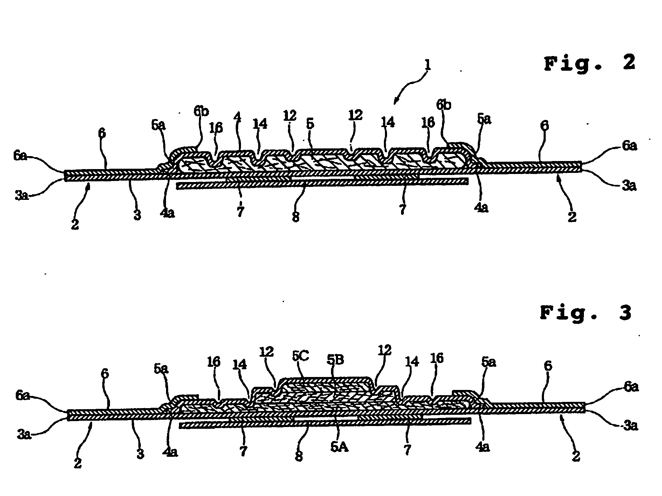

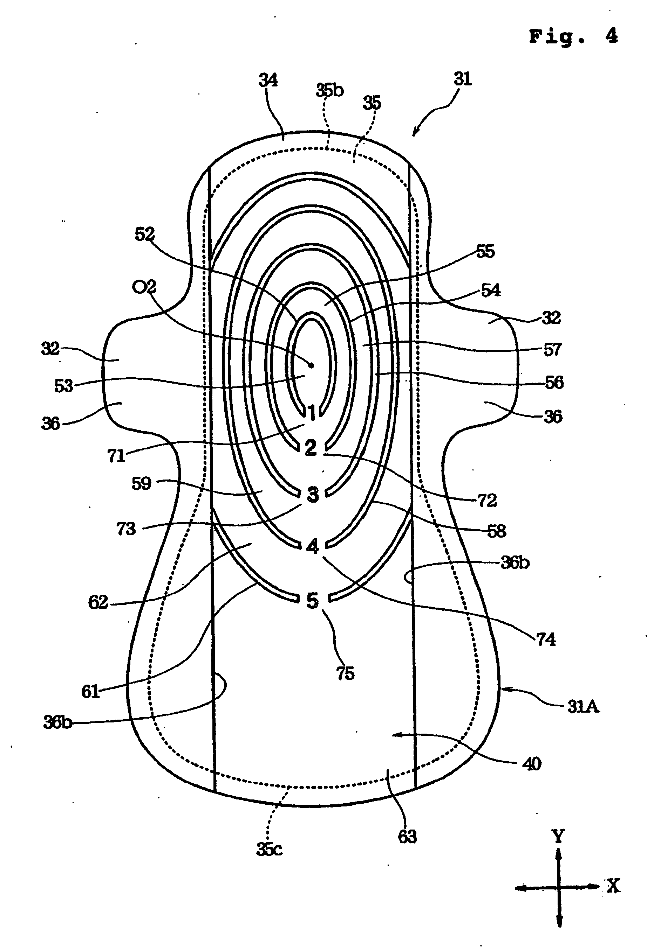

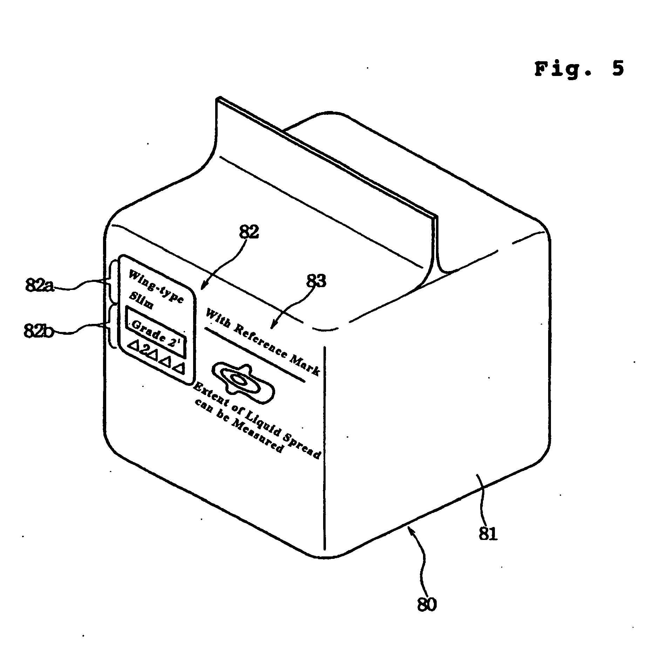

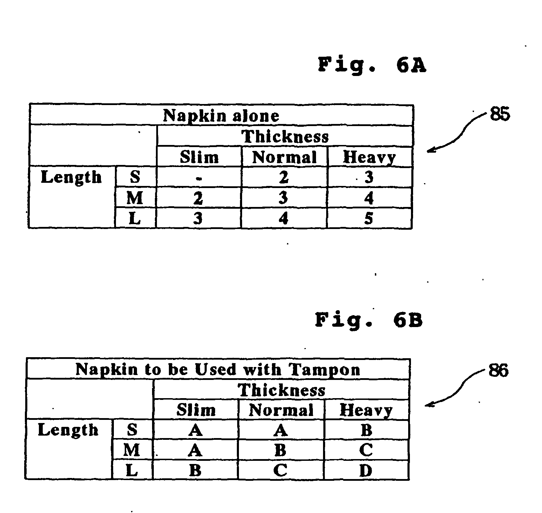

Fig. 1 is a top plan view showing a medium size sanitary napkin 1 of which an absorbent layer is graded " medium" in length and " normal" in thickness; Fig. 2 is a sectional view taken along line II - II of Fig. 1; Fig. 3 is a sectional view which is taken along line II - II as well, but shows another structure; Fig. 4 is a top plan view showing a large size sanitary napkin 31 of which an absorbent layer is graded " large" in length and " normal" in thickness; Fig. 5 is a perspective view showing a package of sanitary napkins having a grade indication thereon; and Figs. 6A and 6B illustrates indications showing relationships between liquid spread grades and size grades of sanitary napkins.

The medium size sanitary napkin 1 of Fig. 1 and the large size sanitary napkin 31 of Fig. 4 are designed to be worn by a female during a menstruation while being attached to an inner side of a crotch portion of an undergarment. The choice of article depends on wearer's menstrual cycle and wearing time. The medium size sanitary napkin 1 of Fig. 1 and the large size sanitary napkin 31 of Fig. 4 have liquid receiving regions where reference marks and grade designators based on common standards are provided.

It should be noted that although the difference in size between the sanitary napkin 1 of Fig. 1 and the sanitary napkin 31 of Fig. 4 is not clear from the drawings, the overall size of the sanitary napkin 31 of Fig. 4 is actually much larger than that of the sanitary napkin 1 of Fig. 1.

As shown in Fig. 1, the medium size sanitary napkin 1 has a generally elliptical portion, of which the upper portion of the drawing is a front end portion and the lower portion of the drawing is a rear end portion to be applied to the buttocks of a wearer's body. As used herein, a Y-direction indicates a longitudinal direction of the napkin, and an X-direction indicates a transverse direction of the napkin. The sanitary napkin 1 is further provided with wing portions 2, 2, which project transversely outwardly from the generally elliptical portion at positions offset toward the front end portion from a transversely extending centerline of the sanitary napkin 1.

As shown in Fig. 2, the sanitary napkin 1 comprises a liquid barrier sheet 3 which is impermeable to liquid and a topsheet 4 which is permeable to liquid. The liquid barrier sheet 3 is of the same top plan shape as the sanitary napkin 1 shown in Fig. 1. That is, the liquid barrier sheet 3 has a generally elliptical portion and left and right projecting portions for forming the wing portions 2, 2. In the topsheet 4, on the other hand, a width (i.e., distance between left and right side edges 4a, 4a) is constant over the entire length thereof. The topsheet 4 is provided over the entire length of the sanitary napkin 1, so that front and rear edges 4b and 4c of the topsheet 4 coincide with front and rear edges of the liquid barrier sheet 3, respectively.

Between the liquid barrier sheet 3 and the topsheet 4, there is disposed an absorbent layer 5. Left and right side edges 5a, 5a of the absorbent layer 5 are inwardly spaced apart from the left and right side edges 4a, 4a of the topsheet 4, respectively. As shown in Fig. 1, front and rear edges 5b, 5c of the absorbent layer 5 are curved. The front edge 5b is inwardly spaced apart from the front edge of the liquid barrier sheet 3 and the front edge 4b of the topsheet 4, and the rear edge 5c is inwardly spaced apart from the rear edge of the liquid barrier sheet 3 and the rear edge 4c of the topsheet 4.

On transversely opposed left and right side portions of the sanitary napkin 1, there are provided liquid impermeable cover sheets 6, 6. These cover sheets 6, 6 have opposing edges 6b, 6b, which are equally spaced apart form a longitudinally extending centerline O - O of the article and positioned slightly inwardly apart form the left and right side edges 5a, 5a of the absorbent layer 5. From the edges 6b, 6b, these cover sheets 6, 6 are extended transversely outwardly beyond the left and right side edges 5a, 5a of the absorbent layer 5 and the left and right side edges 4a, 4a of the topsheet 4, until left and right side edges 6a, 6a which coincide with left and right side edges 3a, 3a of the liquid barrier sheet 3. Thus, the individual wing portions 2, 2 are formed of a laminate of the cover sheet 6 and the liquid barrier sheet 3.

The liquid barrier sheet 3 and the absorbent layer 5 are bonded to each other through a hot-melt type adhesive or the like. The absorbent layer 5 and the topsheet 4 are bonded to each other through a hot-melt type adhesive or the like, which is applied not to impair liquid permeability of the topsheet 4. The cover sheets 6, 6 are bonded to the liquid barrier sheet 3 and the topsheet 4 through a hot-melt type adhesive or the like.

On a garment surface of the liquid barrier sheet 3, as shown in Fig. 2, there are provided pressure sensitive adhesive layers 7, 7 for fixing the article on an inner side of a crotch portion of an undergarment. Before use, these pressure sensitive adhesive layers 7, 7 are covered with a release sheet 8.

The sanitary napkin 1 has a liquid receiving region 10 where the topsheet 4 and the absorbent layer 5 overlap each other. The liquid receiving region 10 has a width W and a length identical to that of the absorbent layer 5, as defined by the opposing edges 6b, 6b of the cover sheets 6, 6 and the front and rear edges 5b, 5c of the absorbent layer 5. That is, the liquid receiving region 10 as used herein means a region in which a discharged liquid given to the body surface of the sanitary napkin 1 can pass through the topsheet 4 for immediate absorption by the absorbent layer 5. Therefore, if a pair of leakage preventing walls is formed of a hydrophobic sheet so as to rise from the body surface of the article at positions transversely equally spaced apart from the centerline O - O, for instance, the liquid receiving region 10 where the topsheet 4 and the absorbent layer 5 overlap each other is defined by the leakage preventing walls.

As shown in Fig. 1, the liquid receiving region 10 is provided with a first reference mark 12, a second reference mark 14 and a third reference mark 16 which are visible from the side of the body surface. A discharged liquid (i.e., menstrual blood) given to the liquid receiving region 10 is absorbed by the absorbent layer 5 through the topsheet 4, and then, two-dimensionally spread in the topsheet 4 and the absorbent layer 5. After use of the sanitary napkin 1, an extent of liquid spread in the liquid receiving region 10 can be measured (graded) by comparing the menstrual blood spread in the liquid receiving region 10 with the reference marks 12, 14 and 16.

The first reference mark 12 is a continuous border line surrounding a first spread region 13 of a predetermined area which is symmetrical about the centerline O - O. The second reference mark 14 is also a continuous border line, but outwardly spaced apart from the first reference mark 12 to surround the first reference mark 12. The region between the first reference mark 12 and the second reference mark 14 is called second spread region 15. Likewise, the third reference mark 16 is a continuous border line which is outwardly spaced apart from the second reference mark 14 to surround the second reference mark 14. The region between the second reference mark 14 and the third reference mark 16 is called third spread region 17. On the other hand, the region surrounding the third reference mark 16 is called outside spread region 18.

All the first reference mark 12, second reference mark 14 and third reference mark 16 surround an arbitrary center point O1 taken on the centerline O - O. In addition, these first reference mark 12, second reference mark 14 and third reference mark 16 are spaced one from another toward the periphery of the liquid receiving region 10 from the center point O1. Here, the center point O1 is provided to coincide with the center of a discharging part (e.g., vaginal opening) of a wearer during wear.

In addition, the liquid receiving region 10 is provided with a first grade designator 21, a second grade designator 22 and a third grade designator 23. The first grade designator 21 is a numerical character " 1" and assigned to the first reference mark 12. The second grade designator 22 is a numerical character " 2" and assigned to the second reference mark 14. The third grade designator 23 is a numerical character " 3" and assigned to the third reference mark 16.

The reference marks 12, 14 and 16 and the grade designators 21, 22 and 23 may be formed in any manner known in the art, as long as they are visible when the sanitary napkin 1 is viewed from the side of the body surface. In this example, as shown in Fig. 2, the reference marks 12, 14 and 16 and the grade designators 21, 22 and 23 are compressed portions formed by heating the absorbent layer 5 and the topsheet 4 under pressure. Such compressed portions can be formed with an embossing roll, on which protrusions are formed in a pattern of the reference marks 12, 14 and 16 and the grade designators 21, 22 and 23. The embossing roll is heated to 120 to 140 °C, and the sanitary napkin 1 is pressed at a pressure of 147 to 196 N.

When the sanitary napkin 1 of Fig. 1 is worn, a menstrual blood given to the liquid receiving region 10 is introduced into the absorbent layer 5 through the topsheet 4, and spread in the topsheet 4 and the absorbent layer 5. After use of the sanitary napkin 1, an extent of menstrual blood spread in the liquid receiving region 10 can be visually measured. More specifically, when a menstrual blood given to the first spread region 13 is spread to reach near the first reference mark 12, the liquid spread can be graded " 1" . When the menstrual blood is spread into the second spread region 15 to reach near the second reference mark 14, the liquid spread can be graded " 2" . When the menstrual blood is spread into the third spread region 17 to reach near the third reference mark 16, the liquid spread is can be graded " 3" . When the menstrual blood is further spread beyond the third reference mark 16 into the outside spread region 18, the liquid spread can be graded more than " 3" . At this time, if the spread menstrual blood does not reach the opposing edges 6b, 6b of the cover sheets 6, 6, the liquid spread may be roughly graded about " 4".

In this example, as shown in Fig. 2, the individual reference marks 12, 14 and 16 are continuous border lines where the topsheet 4 and the absorbent layer 5 are compressed by heating under pressure, so that the density of the absorbent layer 5 is increased more in the reference marks 12, 14 and 16 than in the non-compressed regions. Therefore, when a menstrual blood given to the first spread region 13 is spread to reach the first reference mark 12, then, the menstrual blood readily permeates along the first reference mark 12 where the density of the absorbent layer 5 is increased. Accordingly, the menstrual blood can be prevented from spreading beyond the first reference mark 12 into the second spread region 15 until the menstrual blood is spread over the entire first spread region 13. Such phenomenon occurs in the second reference mark 14 and the third reference mark 16, as well. In other words, the menstrual blood migrates to the second spread region 15 after having spread over the entire first spread region 13, migrates to the third spread region 17 after having spread over the entire second spread region 15, and migrates to the outside spread region 18 after having spread over the entire third spread region 17. With the reference marks 12, 14 and 16 thus formed, an extent of menstrual blood spread can be accurately measured.

Fig. 3 is a sectional view showing another structure of sanitary napkin 1, which constitutes an embodiment of the present invention.

The absorbent layer of Fig. 3 is of a three-layer structure having a lower layer 5A, an intermediate layer 5B and an upper layer 5C. More specifically: in the first spread region 13 surrounded by the first reference mark 12, the absorbent layer is of the three-layer structure; in the second spread region 15 surrounded by the second reference mark 14, the absorbent layer is of a two-layer structure; and in the third spread region 17 surrounded by the third reference mark 16, the absorbent layer is of a single-layer structure.

If the absorbent layer having such three-layer structure is entirely heated under pressure during formation of the reference marks 12, 14 and 16 and the grade designators 21, 22 and 23, the density of the absorbent layer can be varied to satisfy the following relationship: the first spread region 13 > the second spread region 15 > the third spread region 17. With the fiber density of the absorbent layer thus varied, a menstrual blood migrates to the second spread region 15 after the menstrual blood has been sufficiently spread in the first spread region 13 and the absorbent layer has been saturated in the first spread region 13. Then, the menstrual blood reaches the third spread region 17 after the second spread region 15 has been saturated with the menstrual blood thus spread beyond the first reference mark 12. Therefore, an extent of menstrual blood spread can be accurately comprehended with the reference marks.

Moreover, in the case where the absorbent layer is of the three-layer structure, as shown in Fig. 3, the liquid absorption capacity per unit area can be varied to satisfy the following relationship: the first spread region 13 > the second spread region 15 > the third spread region 17. With the liquid absorption capacity per unit area being thus varied, since the time required for the first spread region 13 to be saturated with the menstrual blood is increased, the area of the first spread region 13 can be decreased. Therefore, the number of reference marks, which has to be arranged within the liquid receiving region 10 of a limited area, can be increased.

Next, preferred materials of the individual components of the sanitary napkin 1 will be described.

The liquid barrier sheet 3 and the cover sheet 6 are formed of a liquid impermeable sheet, as exemplified by a resin film comprising at least one of PE (polyethylene), PP (polypropylene), PET (polyethylene terephthalate) and EVA (ethylene-vinyl acetate copolymer). The resin film may be made permeable to moisture (breathable) by adding filler or the like and stretching the film to form fine apertures. In an alternative, a hydrophobic or water-repellent nonwoven fabric may be used.

The topsheet 4 is permeable to liquid. In addition, the topsheet 4 is preferably permeable to a menstrual blood, which has been once absorbed by the absorbent layer 5 through the topsheet 4 and then spread in the absorbent layer 5 beneath the topsheet 4. In order to satisfy the above-mentioned condition, the topsheet 4 may be formed of, for example, a thermal bonded nonwoven fabric having a basis weight of about 25 g/m² and comprising sheath/core bicomponent synthetic fibers, of which the sheath is polyethylene and the core is polyester. In an alternative, the topsheet 4 may be formed of a liquid permeable film, which is a resin film comprising at least one of PE, PP, PET and EVA and formed with a large number of liquid passage holes. This film preferably contains inorganic filler for whitening. In this case, the menstrual blood spread in the absorbent layer 5 can be seen through the liquid passage holes. It is also possible to adjust whiteness of the film by reducing the filler content to such an extent that the menstrual blood spread in the absorbent layer 5 can be seen through it.

The absorbent layer may comprise any of a wide variety of liquid absorbent materials commonly used in absorbent articles. For example, the absorbent layer 5, the lower layer 5A, the intermediate layer 5B and the upper layer 5C are mainly comprised of pulp, in which superabsorbent polymers are dispersed. Here, the absorbent layer is wrapped in hydrophilic tissue paper as a whole.

Next, the large size sanitary napkin 31 of Fig. 4 will be described.

The large size sanitary napkin 31 of Fig. 4 has a rear portion 31A (the lower portion of the drawing), which is transversely outwardly extended so as to function as a so-called " hip guard" for covering a large surface of the buttocks of a wearer. The sanitary napkin 31 is further provided with wing portions 32, 32 projecting transversely outwardly at positions offset forwardly from a transversely extending centerline of the napkin.

The structure of the sanitary napkin 31 of Fig. 4 is similar to that shown in Fig. 2 or 3. In the sanitary napkin 31, an absorbent layer 35 is disposed between a liquid barrier sheet and a liquid permeable topsheet 34. On transversely opposed left and right side portions of the body surface of the sanitary napkin 31, there are provided liquid impermeable cover sheets 36, 36. These components are bonded to each other, similar to those of the medium size sanitary napkin 1.

The sanitary napkin 31 has a liquid receiving region 40, which is defined by opposing edges 36b, 36b of the cover sheets 36, 36 and front and rear edges 35b, 35c of the absorbent layer 35.

The liquid receiving region 40 has a first reference mark 52 about a center point 02, which is to be applied to the center of a discharging part of a wearer's body during wear. The region surrounded by the first reference mark 52 is a first spread region 53. Outside the first reference mark 52, there is provided a second reference mark 54. The region between the first reference mark 52 and the second reference mark 54 is a second spread region 55. Outside the second reference mark 54, there is provided a third reference mark 56. The region between the second reference mark 54 and the third reference mark 56 is a third spread region 57. Outside the third reference mark 56, there is provided a fourth reference mark 58. The region between the third reference mark 56 and the fourth reference mark 58 is a fourth spread region 59. Outside the fourth reference mark 58, there is provided a fifth reference mark 61. The region between the fourth reference mark 58 and the fifth reference mark 61 is a fifth spread region 62. The region outside the fifth reference mark 61 is an outside spread region 63.

In addition, the liquid receiving region 40 is provided with a first grade designator 71, a second grade designator 72, a third grade designator 73, a fourth grade designator 74 and a fifth grade designator 75. The first grade designator 71 is a numerical character " 1" and assigned to the first reference mark 52. Likewise, the second, third, fourth and fifth grade designators 72, 73, 74 and 75 are numerical characters " 2" , " 3" , " 4" and " 5" and assigned to the second, third, fourth and fifth reference marks 54, 56, 58 and 61, respectively.

The reference marks 52, 54, 56, 58 and 61 and the grade designators 71, 72, 73, 74 and 75 shown in Fig. 4 are compressed portions formed by heating the absorbent layer 35 and the topsheet 34 under pressure, similar to those of the sanitary napkin 1.

The reference marks 12, 14 and 16 and the grade designators 21, 22 and 23 of the sanitary napkin 1 and the reference marks 52, 54, 56, 58 and 61 and the grade designators 71, 72, 73, 74 and 75 of the sanitary napkin 31 are provided based on common standards. That is, the amount of liquid absorbed by the absorbent layer 5 when a menstrual blood is spread to the first reference mark 12 of the sanitary napkin 1 is equal to the amount of the liquid absorbent by the absorbent layer 35 when a menstrual blood is spread to the first reference mark 52 of the sanitary napkin 31. At this time, the liquid spread can be graded " 1" in both the sanitary napkin 1 and the sanitary napkin 31. Likewise, the amount of liquid absorbed by the absorbent layer 5 when a menstrual blood is spread to the second reference mark 14 of the sanitary napkin 1 is almost equal to the amount of the liquid absorbent by the absorbent layer 35 when a menstrual blood is spread to the second reference mark 54 of the sanitary napkin 31. At this time, the liquid spread can be graded " 2" in both the sanitary napkin 1 and the sanitary napkin 31. The similar statements are true for grade " 3" .

Hereinabove, only the sanitary napkin 1 of which the absorbent layer 5 is graded " medium" in length and " normal" in thickness and the sanitary napkin 31 of which the absorbent layer 35 is graded " large" in length and " normal" in thickness have been described. However, it is, of course, possible to provide other kinds of sanitary napkins with similar reference marks based on the above-mentioned common standards. By wearing any one of the sanitary napkins having such reference marks, an extent of menstrual blood spread can be measured after use. With this, a wearer can understand relationships between an extent of menstrual blood spread and her menstrual cycle, physical constitution, physique, wearing time and so on. These data can contribute to health care according to her constitution.

Here, the sanitary napkin 1 itself is graded " 3" in liquid spread, which means that if a menstrual blood is given in such an amount as to spread beyond the third reference mark 16, a fear of lateral leakage will be caused. On the other hand, the sanitary napkin 31 itself is graded " 4" in liquid spread, which means that if a menstrual blood is given in such an amount as to spread beyond the fourth reference mark 58, a fear of lateral leakage will be caused. Such fear of lateral leakage can be dispelled by choosing a sanitary napkin of an appropriate liquid spread grade according to her physical condition at the time, physical constitution and so on.

For example, if the large size sanitary napkin 31 of Fig. 4 is worn for a predetermined time and then the liquid spread is graded " 3" , a wearer can understand that the sanitary napkin 1 of Fig. 1 will be more suitable for the same wearing time if she is in the same physical condition at the time of next use. That is, she can understand that the large size sanitary napkin 31 of Fig. 4 is no more necessary.

Conversely, if the medium size sanitary napkin 1 of Fig. 1 is worn for a predetermined time and then it is found that a menstrual blood is widely spread beyond the third reference mark 16 to the outside spread region 18, a wearer can understand that it is not optimal to choose the sanitary napkin 1 of Fig. 1 for the same wearing time if she is in the same physical condition at the time of next use, but it is desirable to choose the large size sanitary napkin 31 of Fig. 4.

Once a user has understood relationships between an extent of liquid spread and her menstrual cycle, physical constitution, wearing time and so on, by wearing sanitary napkins having the common reference marks for a certain period, it is not necessarily required to use these sanitary napkins having the reference marks, and the user can choose an optimal one even if sanitary napkins do not have the reference marks, as long as their liquid spread grades are obvious.

For example, if a user wants to use a sanitary napkin having a liquid spread grade of " 3" , she can choose one having the same size (length and thickness) as the sanitary napkin 1. This sanitary napkin is not required to have the reference marks 12, 14 and 16 and the grade designators 21, 22 and 23.

Fig. 5 is a perspective view showing a package 80 of sanitary napkins. Sanitary napkins are folded on two or three fold lines and individually packaged. These individually packaged sanitary napkins are stacked one on another and packaged in a packaging sheet 81 formed of a resin film or the like, thereby providing the package 80.

On the packaging sheet 81, a product description 82 is printed to appear on the package 80, together with an ornamental pattern, a trade name and so on (not shown). The product description 82 includes captions 82a indicating the kind of sanitary napkins. In Fig. 5, these captions 82a include the word" Wing-type" indicating that sanitary napkins having wing portions are accommodated and the word " Slim" indicating that the absorbent layer of the accommodated sanitary napkin is of a thin type.

The product description 82 also includes a grade indication 82b teaching the liquid spread grade of the sanitary napkins. From Fig. 5, it is seen that the liquid spread grade of the accommodated sanitary napkins is " 2" .

On the packaging sheet 81, moreover, a description 83 is printed to appear on the package 80, teaching that the accommodated sanitary napkins have reference marks similar to those of Figs. 1 and 4.

Here, the grade indication 82b teaching the liquid spread grade of sanitary napkins may be printed on the packaging sheet 81, as a part of the product description 82. In this case, it is preferred that an instruction such as " Suitable Liquid Spread Grade can be Found with Sanitary Napkin having Reference Marks" is printed together with the grade indication 82b.

It is also possible that only one or a few of the accommodated sanitary napkins have the reference marks and the grade designators and the remaining sanitary napkins have neither reference marks nor grade designators.

Commonly, sanitary napkins of the same trade name are variously graded for sale as to size (length and thickness) of the absorbent layer. In this case, an indication 85 is preferably printed on the exterior surface of a packaging sheet in which sanitary napkins of a single size grade are packaged. As shown in Fig. 6A, the indication 85 is a table showing relationships between size grades and liquid spread grades.

In the indication 85 of Fig. 6A, sanitary napkins are divided into " small" , " medium" and " large" depending on the length of the absorbent layer, and subdivided into " slim" , " normal" and " heavy" depending on the thickness of the absorbent layer. From Fig. 6A, it is seen that a sanitary napkin of which the absorbent layer is graded " small" in length and graded " slim" in thickness does not exist, so that this product is classified into 8 size grades. In the indication 85, the liquid spread grades are indicated at " 1" , " 2" , " 3" , " 4" and " 5" for the individual size grades of the product. Once a user has understood relationships between an extent of liquid spread and her menstrual cycle, wearing time and so on, by wearing sanitary napkins having reference marks similar to those of Figs. 1 and 4 for a certain period, she can certainly choose a product suitable for use with reference to the indication 85.

The grading as to liquid spread is also applicable to a napkin to be used in combination with a tampon. This napkin is to be worn during use of a tampon, so as to absorb a menstrual blood leaking out of the vaginal opening. If this napkin to be used in combination with a tampon is provided with reference marks and grade designators similar to those of Figs. 1 and 4, a suitable product can be chosen according to the measured extent of liquid spread.

Fig. 6B illustrates an indication 86 to be printed on a packaging sheet in which napkins to be used in combination with tampons are packaged. In the indication 86, sanitary napkins to be used in combination with tampons are divided into " small" , " medium" and " large" depending on the length of the absorbent layer, and subdivided into " slim" , " normal" and " heavy" depending on the thickness of the absorbent layer, so that the product is classified into total 9 size grades. In addition, the liquid spread grades are indicated at " A" , " B" , " C" and " D" for the individual size grades of the product. If the napkin to be used in combination with a tampon is provided with reference marks and grade designators similar to those of Fig. 1 and used for a certain period, a user can understand relationships between an extent of liquid spread and her menstrual cycle, wearing time and so on. Subsequently, she can certainly use an optimal napkin in combination with a tampon by choosing a product with reference to the indication 86 of Fig. 6B.

It should be noted that the reference marks and the grade designators provided in the liquid receiving region may be in any form, as long as they are visible from the side of the body surface.

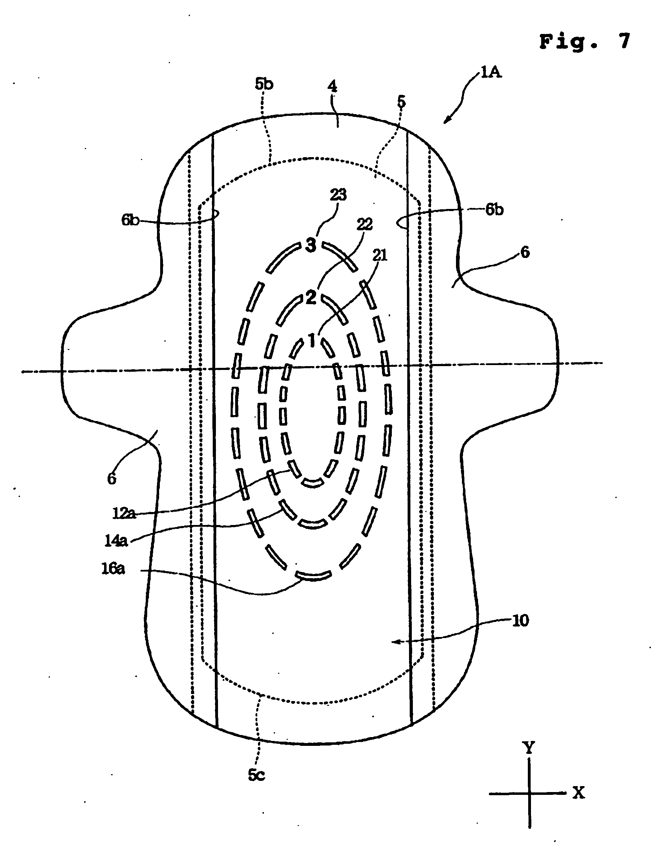

Fig. 7 shows a sanitary napkin 1A which is different only in the form of reference marks from the sanitary napkin 1 of Fig. 1. In the sanitary napkin 1A, a first reference mark 12a, a second reference mark 14a and a third reference mark 16a are discontinuous (interrupted) border lines each surrounding a predetermined area. These discontinuous border lines are compressed portions formed by heating the absorbent layer 5 and the topsheet 4 under pressure. Here, the reference marks 12, 14, 16, 12a, 14a and 16a and the grade designators 21, 22, 23 may be formed by heating only the topsheet 4 under pressure, as a pattern of the topsheet **4**.

It should also be noted that the numerical grade designators 21, 22, 23, 71, 72, 73, 74 and 75 are not necessarily required. Even without these grade designators, a user can understand that the region inside the first reference mark is the first spread region and the region outside the first reference mark is the second spread region.

In the foregoing embodiments, moreover, the body surface of the topsheet may be embossed such that the spread regions have different patterns. For example, it is possible that the first spread region is of flower patterns and the second spread region is of leaf patterns. By providing different patterns for different spread regions, the spread regions can be clearly distinguished from each other.

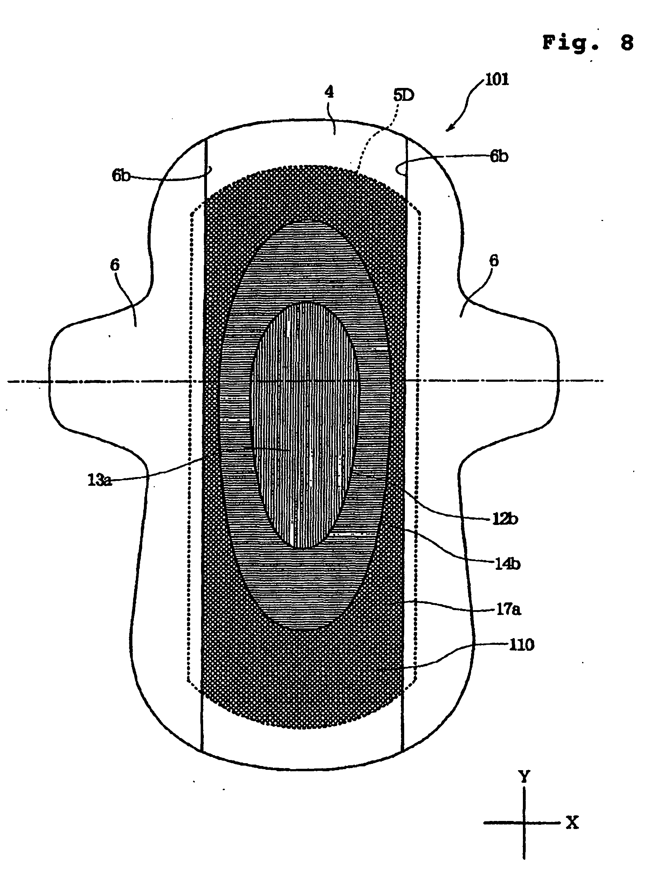

Fig. 8 shows a sanitary napkin 101, which has the same basis structure as the sanitary napkin of Fig. 1, but its overall size is slightly smaller than that of the sanitary napkin of Fig. 1.

The sanitary napkin 101 has a liquid receiving region 110, which is defined by the opposing edges 6b, 6b of the cover sheets 6, 6 and in which an absorbent layer 5D is present. The liquid receiving region 110 has a first spread region 13a at the center thereof, a second spread region 15a surrounding the first spread region 13a, and an outside spread region 17a surrounding the second spread region 15a, and these spread regions 13a, 15a and 17a are of different colors as viewed from the side of the body surface of the napkin. For example, the first spread region 13a is a red or pink color, the second spread region 15a is a blue color, and the outside spread region 17a is an orange color. Here, the boundary between the red or pink color and the blue color is a first reference mark 12b, and the boundary between the blue color and the orange color is a second reference mark 14b. Compressed portions similar to those of Fig. 2 may or may not be formed along the first reference mark 12b and the second reference mark 14b.

These colors may be provided by directly coloring the topsheet 4 formed of a nonwoven fabric or a resin film having liquid passage holes. In an alternative, the absorbent layer 5D may be colored. In this case, for example, the absorbent layer 5D is of a three-layer structure comprising an orange-colored lower layer, a blue-colored intermediate layer which has a periphery coinciding with the second reference mark 14b and is laid on the orange-colored lower layer, and a red or pink-colored upper layer which has a periphery coinciding with the first reference mark 12b and is laid on the blue-colored intermediate layer, so that the orange, blue and red or pink colors can be seen through the topsheet 4 covering the absorbent layer 5D.

It should be noted that the reference marks and the grade designators of Figs. 1, 4 and 7 may be printed on the topsheet. In this case, the topsheet is preferably formed of a resin film having liquid passage holes.

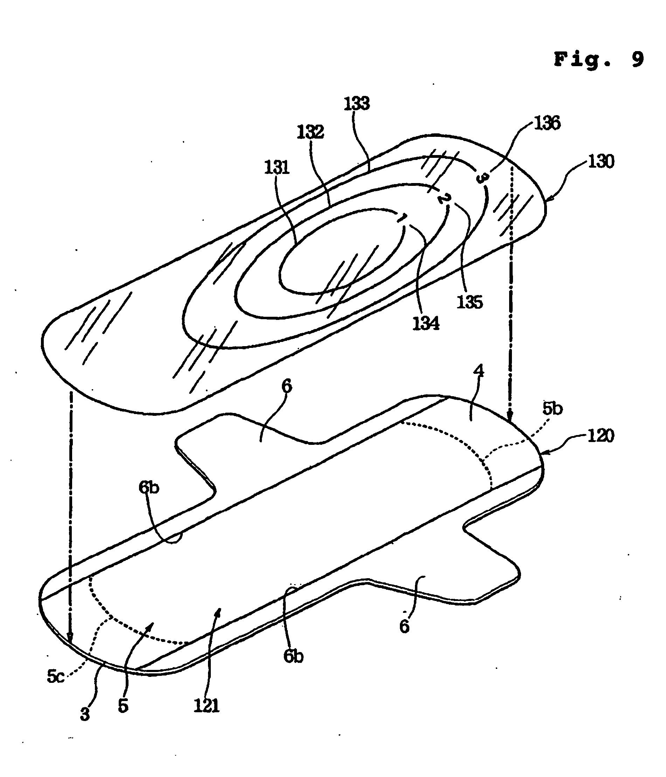

Fig. 9 is a perspective view showing an example which does not constitute an embodiment of the present invention.

The sanitary napkin of Fig. 9 comprises a napkin body 120 and a measuring sheet 130. The napkin body 120 has the same basis structure as the sanitary napkin 1 of Fig. 1. The napkin body 120 has a liquid receiving region 121, which is defined by the opposing edges 6b, 6b of the cover sheets 6, 6 and the front and rear edges 5b, 5c of the absorbent layer 5. In this embodiment, however, the reference marks and the grade designators are not provided in the liquid receiving region 121.

The measuring sheet 130 is formed of a see-through sheet material such as a transparent film. The measuring sheet 130 is of the same size as the generally elliptical portion of the napkin body 120. In an alternative, the measuring sheet 130 may be of the same size as the entire napkin body 120. In addition, a first reference mark 131, a second reference mark 132 and a third reference mark 133 are printed on the measuring sheet 130, together with a first grade designator 134, a second grade designator 135 and a third grade designator 136.

After wearing the napkin body 120 for a certain period, the measuring sheet 130 is put on the body surface of the napkin body 120. By comparing a liquid spread in the liquid receiving region 121 with the reference marks 131, 132 and 133, a user can grade the spread liquid.

These reference marks 131, 132 and 133 on the measuring sheet 130 are provided based on standards that are common to the reference marks 12, 14 and 16 provided in the liquid receiving region 10 of the sanitary napkin 1. Therefore, when an equal amount of liquid is given to the sanitary napkins 1 and 31 and the napkin body 120, the same liquid spread grade can be obtained.

Here, a single napkin body 120 may be individually packaged together with a single measuring sheet 130. In an alternative, a single measuring sheet 130 may be accommodated in a package similar to that of Fig. 5, in which a plurality of individually packaged napkin bodies 120 are packaged.

The reference mark should not be limited to the border line surrounding a predetermined area as shown in Fig. 1. For example, short reference marks linearly extending in the transverse direction (X-direction) or curved short reference marks extending in the transverse direction may be provided in the liquid receiving region 10 of the sanitary napkin 1 of Fig. 1, in spaced relationship to each other in the longitudinal direction (Y-direction).

It should be noted that the absorbent article of the present invention is not limited to the sanitary napkin, but includes vaginal discharge absorbing sheet, urine absorbing pad, disposable diaper and the like. With the reference marks of the present invention, a user can choose suitable one from a variety of vaginal discharge absorbing sheets of different thicknesses in accordance with her wearing time, physical condition and so on. Also in the case of urine absorbing pads and disposable diapers, a user can choose suitable one in accordance with his/her physique, age, physical constitution and so on.

In the case of vaginal discharge absorbing sheets, the liquid receiving region is preferably colored black or navy blue to enhance the contrast between the vaginal discharge and the liquid receiving region, so that an extend of spread of the vaginal discharge in the liquid receiving region can easily be visually measured.

In the case of urine absorbing pads and disposable diapers, the topsheet may be formed of a material, such as spunbonded nonwoven fabric comprising polypropylene fibers, which assumes a translucent color when wetted, and the reference marks may be provided between the topsheet and the absorbent layer. In this case, the reference marks can be seen through the topsheet only within an area wetted with a liquid.

As has been described hereinabove, a discharge amount of liquid, which varies depending on user's physical constitution, physical condition and so on, can be measured immediately after use of the absorbent article of the present invention, which contributes to health care and so on. In addition, a user can choose a suitable absorbent article for next use.

Once the discharge amount has been measured using the reference mark of the absorbent article, moreover, since the package of absorbent articles has the grade indication, a user can easily choose a suitable product with reference to the grade indication.

## Claims

1. An absorbent article comprising: a liquid permeable topsheet disposed on a body surface of the article; a liquid barrier sheet for preventing liquids from exuding toward a garment surface of the article; and an absorbent layer disposed between the topsheet and the liquid barrier sheet, the absorbent article having a liquid receiving region of a predetermined area where the topsheet and the absorbent layer overlap each other, wherein first, second and third reference marks, for visually measuring an extent of liquid spread in the liquid receiving region, are provided on a body surface of the topsheet,
the reference marks comprise border lines where the topsheet and absorbent layer are compressed, by heating under pressure, and the density of the absorbent layer is increased in the compressed regions,
the absorbent layer is a three-layer structure having a lower layer, an intermediate layer and an upper layer,
the first reference mark surrounds a first spread region in which the absorbent layer is of the three-layer structure, the second reference mark surrounds a second spread region in which the absorbent layer is of a two-layer structure, and the third reference mark surrounds a third spread region in which the absorbent layer is of a single-layer structure.

2. The absorbent article as set forth in Claim 1, wherein the reference marks are continuous or discontinuous border lines each surrounding a predetermined area of the liquid receiving region.

3. The absorbent article as set forth in Claim 1, wherein grade designators assigning grades to the reference marks are provided on the body surface of the topsheet along with the reference marks.

4. The absorbent article as set forth in Claim 1, wherein the body surface of the topsheet is colored or patterned differently between a region surrounded by each reference mark and a region surrounding each reference mark.

## Patentansprüche

1. Absorbierender Artikel, der Folgendes umfasst: eine flüssigkeitsdurchlässige oberste Schicht, die auf einer Körperfläche des Artikels angeordnet ist, eine Flüssigkeitsbarriereschicht zum Vermeiden, dass Flüssigkeiten in Richtung einer Kleidungsfläche des Artikels exsudieren, und eine absorbierende Schicht, die zwischen der obersten Schicht und der Flüssigkeitsbarriereschicht angeordnet ist, wobei der absorbierende Artikel einen flüssigkeitsaufnehmenden Bereich einer vorbestimmten Region hat, wobei sich die oberste Schicht und die absorbierende Schicht überschneiden, wobei die erste, zweite und dritte Referenzmarke zum visuellen Messen des Ausmaßes der Flüssigkeitsausbreitung im flüssigkeitsaufnehmenden Bereich auf einer Körperfläche der obersten Schicht bereitgestellt werden,
wobei die Referenzmarke Grenzlinien umfasst, worin die oberste Schicht und die absorberende Schicht komprimiert sind, indem sie unter Druck erhitzt werden, und die Dichte der absorbierenden Schicht in den komprimierten Bereichen erhöht wird,
wobei die absorbierende Schicht eine dreischichtige Struktur ist, die eine niedrigere Schicht, eine Zwischenschicht und eine obere Schicht hat,
wobei die erste Referenzmarke einen ersten Spreizbereich umgibt, in dem die absorbierende Schicht eine dreischichtige Struktur ist, und die zweite Referenzmarke einen zweiten Spreizbereich umgibt, in dem die absorbierende Schicht eine zweischichtige Struktur ist, und die dritte Referenzmarke einen dritten Spreizbereich umgibt, in dem die absorbierende Schicht eine einschichtige Struktur ist.

2. Absorbierender Artikel nach Anspruch 1, wobei die Referenzmarken kontinuierliche oder diskontinuierliche Randlinien sind, von denen jede eine vorbestimmte Region des flüssigkeitsaufnehmenden Bereichs umgibt.

3. Absorbierender Artikel nach Anspruch 1, wobei die Stufenbezeichnungen den Referenzmarken Stufen zuordnen, die an der Körperfläche der obersten Schicht zusammen mit den Referenzmarken bereitgestellt wird.

4. Absorbierender Artikel nach Anspruch 1, wobei die Körperfläche der obersten Schicht zwischen einem Bereich, der von jeder Referenzmarke umgeben ist, und einem Bereich, der jede Referenzmarke umgibt, unterschiedlich gefärbt oder gemustert ist.

## Revendications

1. Article absorbant comportant : une feuille de dessus perméable aux liquides disposée sur une surface côté corps de l'article ; une feuille anti-liquides destinée à empêcher les liquides de suinter vers une surface côté vêtement de l'article ; et une couche absorbante disposée entre la feuille de dessus et la feuille anti-liquides, l'article absorbant ayant une région recevant les liquides d'une zone prédéterminée où la feuille de dessus et la couche absorbante se chevauchent l'une par rapport à l'autre, dans lequel des première, seconde et troisième marques de référence, permettant de mesurer visuellement une étendue de dispersion des liquides dans la région recevant les liquides, sont mises en oeuvre sur une surface côté corps de la feuille de dessus,
les marques de référence comportent des lignes de limite où la feuille de dessus et la couche absorbante sont comprimées, par chauffage sous pression, et la densité de la couche absorbante est augmentée dans les régions comprimées,
la couche absorbante est une structure à trois couches ayant une couche inférieure, une couche intermédiaire et une couche supérieure,
la première marque de référence entoure une première région de dispersion dans laquelle la couche absorbante fait partie de la structure à trois couches, la seconde marque de référence entoure une seconde région de dispersion dans laquelle la couche absorbante fait partie d'une structure à deux couches, et la troisième marque de référence entoure une troisième région de dispersion dans laquelle la couche absorbante fait partie d'une structure à une seule couche.

2. Article absorbant selon la revendication 1, dans lequel les marques de référence sont des lignes de limite continues ou discontinues entourant chacune une zone prédéterminée de la région recevant les liquides.

3. Article absorbant selon la revendication 1, dans lequel des indicateurs de classement attribuant des classements aux marques de référence sont mis en oeuvre sur la surface côté corps de la feuille de dessus le long des marques de référence.

4. Article absorbant selon la revendication 1, dans lequel la surface côté corps de la feuille de dessus est en couleur ou à motif de manière différente entre une région entourée par chaque marque de référence et une région entourant chaque marque de référence.
